# EUROPEAN PATENT SPECIFICATION

(11) **EP 4 426 220 B1**
(45) Date of publication and mention of the grant of the patent: **14.10.2026**
(21) Application number: 22790062.8
(22) Date of filing: 30.09.2022
(51) Int. Cl.: A61B 34/10, A61F 9/007, A61B 34/00, A61F 9/008

(54) **COMPUTERIZED TREATMENT PLAN WITH ALTERNATIVES FOR GUIDING OPHTHALMIC SURGERY**
COMPUTERISIERTER BEHANDLUNGSPLAN MIT ALTERNATIVEN ZUR FÜHRUNG VON AUGENOPERATIONEN
PLAN DE TRAITEMENT INFORMATISÉ COMPORTANT DES ALTERNATIVES POUR GUIDER UNE CHIRURGIE OPHTALMIQUE

(30) Priority: 03.11.2021 US 202163275309 P; 02.08.2022 US 202263394520 P
(43) Date of publication of application: 11.09.2024
(73) Proprietor: Alcon Inc., 1701 Fribourg (CH)
(72) Inventor: RAPOPORT, Tobias Jura, 14513 Teltow (DE); KASHANI, Pooria Sharif, Lake Forest, California 92630 (US); GERENA, Carlos, Lake Forest, California 92630 (US); LEE, Sang Yeop, Fullerton, California 92832 (US); PETTIT, George Hunter, Fort Worth, Texas 76134 (US); CAMPIN, John Alfred, Fort Worth, Texas 76134 (US)
(74) Representative: Murgitroyd & Company
(86) International application number: PCT/IB2022/059367
(87) International publication number: WO 2023/079384

(56) References cited:
- EP-A1- 3 305 173

## Description

### TECHNICAL FIELD

The present disclosure relates generally to methods for computerized guidance during performance of ophthalmic surgery, such as the placement of intraocular lenses (IOL) for the treatment of cataracts and other conditions.

### BACKGROUND

Light received by the human eye, passes through the transparent cornea covering the iris and pupil of the eye. The light is transmitted through the pupil and is focused by a crystalline lens positioned behind the pupil in a structure called the capsular bag. The light is focused by the lens onto the retina, which includes rods and cones capable of generating nerve impulses in response to the light.

Through age or disease, the crystalline lens may become cloudy, a condition known as a cataract. Cataracts, and other conditions, are readily treated by removing the crystalline lens and inserting an artificial lens, known as an intraocular lens (IOL). The IOL may be fabricated to additionally correct for aberrations of the patient's eye, such as astigmatism. Inasmuch as astigmatism is the result of asymmetry of the eye, the IOL must be aligned with the asymmetry of the eye in order to compensate for it. The IOL is therefore provided with markers, such as rows of dots at the perimeter of the IOL, which define an axis that may be used to align the IOL. The IOL may be implemented as a toric IOL, which includes spring-like arms, known as haptics, that hold the IOL in place within the capsular bag.

Ophthalmic surgery, such as the placement of an IOL, requires thorough treatment planning beforehand. The treatment plan may be presented to the surgeon during surgery as a printout or as digital image guidance. For example, the treatment plan may include guides superimposed on an image of the patient's eye captured using a digital microscope.
Reference is made to the document EP3305175 which has been cited as exemplary of the background state of the art. EP3305175 relates to a surgical system and image processing method that includes acquiring tomographic images and recognizing predetermined parts of the eye in those images for guiding ophthalmic surgery.

### BRIEF SUMMARY

The scope of the invention is in accordance with the appended claims.

The present disclosure relates generally to a system creating and presenting a primary treatment plan and one or more backup plans for ophthalmic surgery.

Particular embodiments disclosed herein provide a method and corresponding apparatus for guiding ophthalmic surgery. The method includes loading, by a computing device, two or more ophthalmic surgical treatment plans ("treatment plans") having a common pre-operative image of a patient, the two or more treatment plans being different from one another. A live video of an eye of the patient is received and registration of the live video with respect to the pre-operative image is initiated. The method further includes initiating, by the computing device, on a display device coupled to the computing device, presentation of one or more first steps of a first treatment plan of the two or more treatment plans, the one or more first steps defining superimposition of one or more first guides on the live video. The method includes receiving, by the computing device, an instruction to switch to a second plan of the two or more treatment plans. In response to the instruction, the method includes initiating, by the computing device, on the display device, presentation of one or more second steps of a second plan of the two or more treatment plans, the one or more second steps defining superimposition of one or more second guides on the live video, the one or more second guides being different from the one or more first guides.

The following description and the related drawings set forth in detail certain illustrative features of one or more embodiments.

### BRIEF DESCRIPTION OF THE DRAWINGS

The appended figures depict certain aspects of the one or more embodiments and are therefore not to be considered limiting of the scope of this disclosure.
**FIG. 1** illustrates an interface for creating a primary treatment plan and one or more backup plans, in accordance with certain embodiments.
**FIG. 2A** illustrates an incision guide.
**FIG. 2B** illustrates a rhexis guide.
**FIG. 2C** illustrates limbal release incision (LRI) guide.
**FIG. 2D** illustrates a centration guide.
**FIG. 2E** illustrates an alignment guide for a toric IOL.
**FIG. 3** is a process flow diagram of a method for creating a primary treatment plan and one or more backup plans, in accordance with certain embodiments.
**FIG. 4** illustrates a treatment plan hierarchy, in accordance with certain embodiments.
**FIG. 5** illustrates an interface for presenting a primary treatment plan and one or more backup plans, in accordance with certain embodiments.
**FIG. 6** is a process flow diagram of a method for presenting a primary treatment plan and one or more backup plans, in accordance with certain embodiments.
**FIG. 7** illustrates an example computing device that implements, at least partly, one or more functionalities of creating and presenting a primary treatment plan and one or more backup plans.

To facilitate understanding, identical reference numerals have been used, where possible, to designate identical elements that are common to the drawings. It is contemplated that elements and features of one embodiment may be beneficially incorporated in other embodiments without further recitation.

### DETAILED DESCRIPTION

In prior approaches to performing ophthalmic surgery, a single treatment plan is created and loaded into a computing device, such as a digital microscope. If a change in treatment plan is needed, either (a) the surgeon must simply implement the change without the guidance of a treatment plan or (b) the surgery must be paused to load a new treatment plan into the computing device. Where a new treatment plan is loaded, initial steps of the new plan will need to be repeated, such as registering live images of the patient's eye with a pre-operative image of the new plan.

Particular embodiments of the present disclosure provide a primary treatment plan and one or more backup plans that are loaded into a computing device prior to a surgical procedure. If a foreseeable event corresponding to one of the one or more backup plans occurs during the surgical procedure, the backup plan may be selected and presented without the need to reload a new treatment plan onto the computing device.

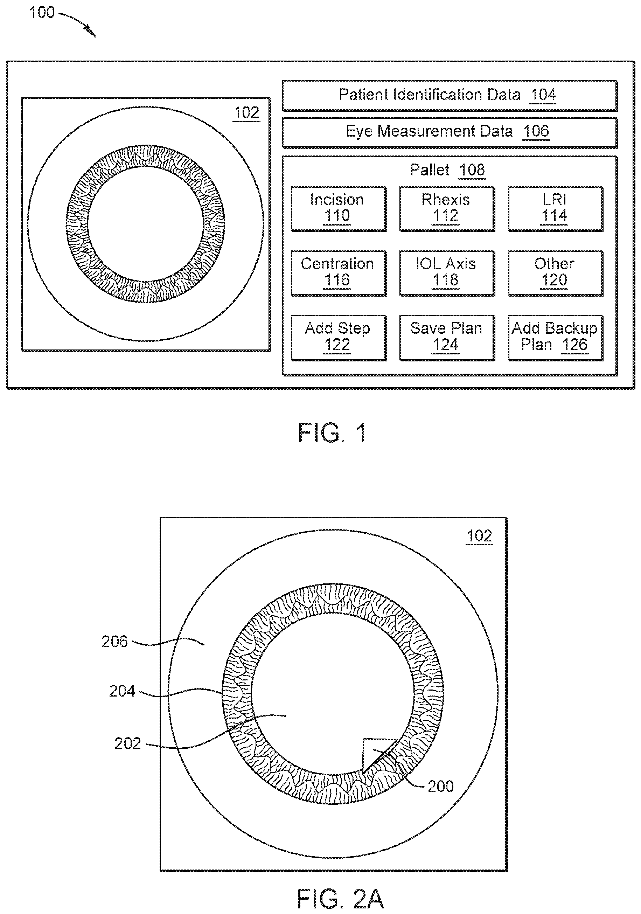

**FIG. 1** illustrates an interface 100 for creating a primary treatment plan and one or more backup plans for performing ophthalmic surgery. The examples presented below describe surgical procedures including replacing the crystalline lens of a patient with an IOL. As an example, a primary treatment plan may include a treatment plan for placing and aligning a toric IOL and backup plans may address contingencies such as bag rupture, patient decision to switch to a different IOL, or other contingencies. The implementations described herein may also be applied to other types of ophthalmic surgery.

The interface 100 may present patient information to assist a user in creating the primary treatment plan and the one or more backup plans. The patient information may include a pre-operative image 102. The pre-operative image 102 may be an image of the patient's eye captured using an imaging device, such as a pre-operative imaging device, examples of which include an optical coherence tomography (OCT), a rotating camera (e.g., a Scheimpflug camera), a magnetic resonance imaging (MRI) device, a keratometer, an ophthalmometer, an optical biometer, or any other digital imaging device. The pre-operative image 102 may be captured prior to creating the primary treatment plan and the one or more backup plans. The patient information may include patient identification data 104, such as patient's name, demographic attributes, photograph, or other identification data. The patient information may include eye measurement data 106. The eye measurement data 106 may include keratometry, biometry, refraction measurement (spherical aberration, cylindrical aberration, astigmatism), and measurements of anatomy of the eye (e.g., limbus diameter). The eye measurement data 106 may include data describing incisions to be made, such as a radius and angle for each of a primary incision and one or more secondary incisions. The eye measurement data 106 may include data describing limbal release incisions (LRI), such as in the form of a diameter and angular extent of each LRI.

The interface 100 may include a pallet 108 enabling the selection of guides to be placed with respect to the pre-operative image 102. The pallet 108 may be embodied as, e.g., a grouping of selectable icons, a menu, a picklist, or any other user interface element known in the art. The pallet 108 may include an incision element 110, rhexis element 112, LRI element 114, centration element 116, IOL axis element 118, and possibly one or more other elements 120. A user may interact with each of the above-described elements by means of clicking with a pointing device (mouse, trackpad), tapping on a touch sensitive element (touch screen, trackpad), making a predefined gesture on the touch sensitive element, inputting a keystroke, voice command, pressing a foot pedal, or any input means known in the art.

Referring to **FIG. 2A****,** while still referring to **FIG. 1****,** the incision element 110, when selected by a user, may enable a user to select a location on the pre-operative image 102 for placing an incision guide 200. For example, the pre-operative image 102 may include representations of the cornea 202, limbus 204, and sclera 206 of the patient's eye. The user may therefore place incision guide 200 at a location in the cornea near the limbus. The user may place multiple incision guides 200, such as a primary incision guide 200 and one or more secondary incision guides 200.

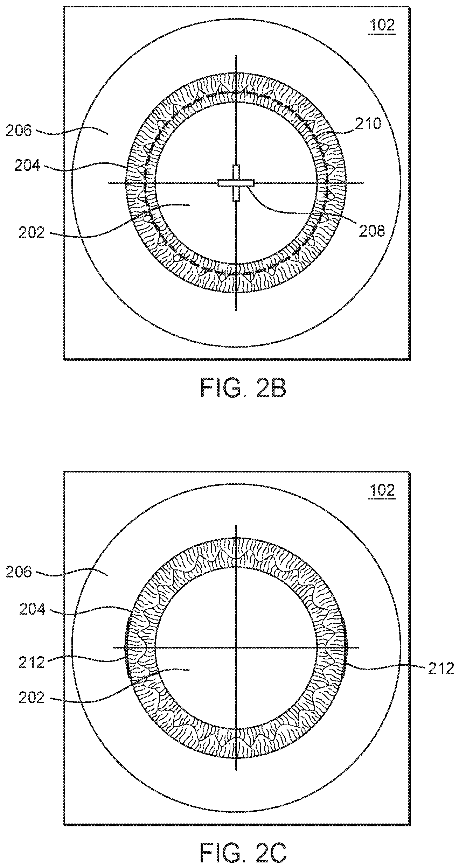

Referring to **FIG. 2B****,** while still referring to **FIG. 1****,** the rhexis element 112, when selected by a user, may enable a user to select a location on the pre-operative image 102 for placing a center 208 of a rhexis and placing a perimeter 210 of the rhexis, e.g., a circle centered on the center 208 and having a diameter specified by the user. The rhexis is an opening in the capsular bag that is formed to enable removal of the crystalline lens and placement of an IOL.

Referring to **FIG. 2C****,** while still referring to **FIG. 1****,** the LRI element 114, when selected by a user, may enable a user to select a location on the pre-operative image for placing one or more LRI guides 212. The LRI guides 212 define placement of limbal release incisions (LRI) and may be placed along the limbus 204 of the eye. The interface 100 may automatically identify the limbus 204, such as using machine learning or other automated image processing approach. Accordingly, the interface 100 may receive an angular position and extent of the LRI on the limbus 204 from a user upon selection of the LRI element 114.

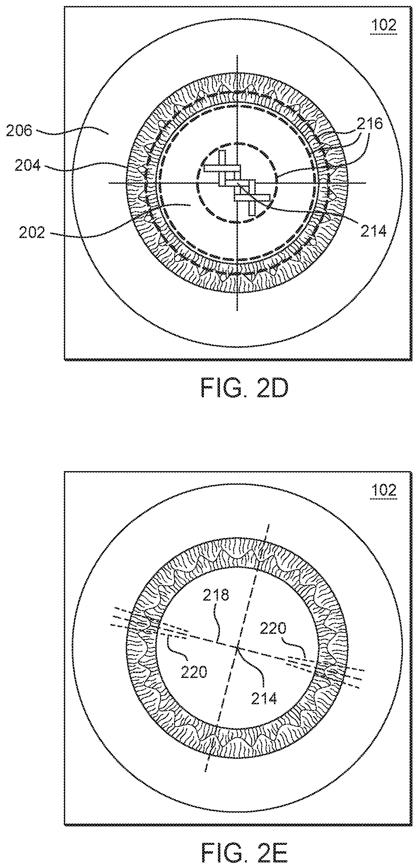

Referring to **FIG. 2D****,** while still referring to **FIG. 1****,** the centration element 116, when selected by a user, may enable a user to select a location on the pre-operative image for placing a center 214 of a centration guide and one or more rings 216 centered on the center 214. For example, the rings 216 may define a desired position of the perimeter of an IOL. A pair of rings 216 may be placed with the gap between the rings 216 corresponding to the desired position of the perimeter of the IOL.

Referring to **FIG. 2E****,** while still referring to **FIG. 1****,** the IOL axis element 118, when selected by a user, may enable a user to select a location on the pre-operative image for placing an IOL axis 218. The IOL axis 218 may be a line passing through the center 214 defined for the centration guide or may use a separately defined center. The IOL axis 218 defines a desired alignment of a toric IOL having asymmetric properties for the treatment of astigmatism. The IOL axis 218 represents a desired alignment of marking dots on the toric IOL. The interface may further automatically place protractors 220 indicating angular measurements to either side of the IOL axis 218 or place and size the protractors 220 in response to inputs from the user following selection of the IOL axis element 118.

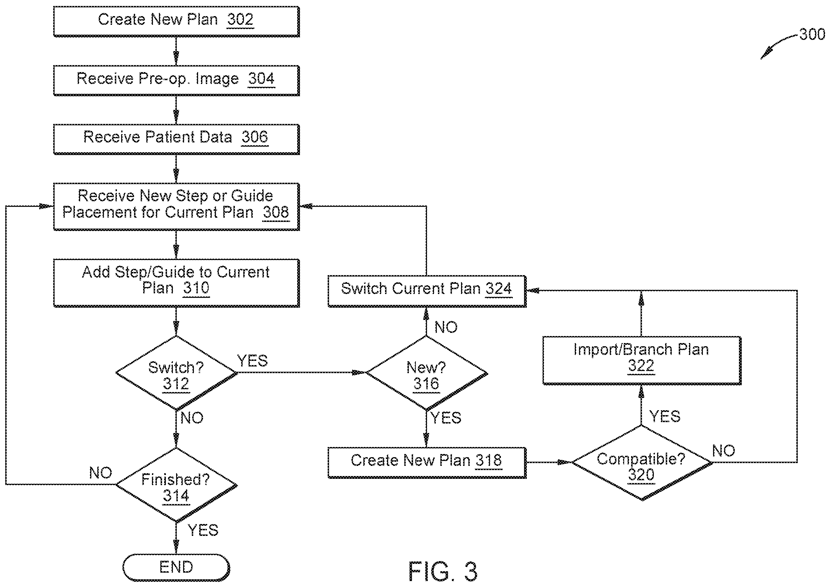

**FIG. 3** illustrates a method 300 that may be executed by a computing device using inputs received through the interface 100 or other interface in order to create a primary treatment plan and one or more backup plans. The method 300 may include creating, at step 302, a new plan. This may include creating a primary treatment plan in the form of a data object, file, or other data structure to store data defining the primary treatment plan. The primary treatment plan may be part of a plan hierarchy to which one or more backup plans may also be added. The plan hierarchy created at step 302 may be populated using the interface 100 or other interface in subsequent steps of the method 300 as described below, including populating the primary treatment plan and one or more backup plans.

The method 300 may include receiving, at step 304, a pre-operative image 102 and receiving, at step 306, patient data, such as patient identification data 104 and eye measurement data 106 as described above. The pre-operative image 102 may be added to the plan hierarchy and be used for the primary treatment plan and one or more backup plans of the plan hierarchy. As an alternative, a backup plan of the one or more backup plans may use a different pre-operative image 102.

The subsequent steps of the method 300 are described below with respect to a "current plan," which may be understood as whichever of the primary treatment plan and one or more backup plans is currently being modified by a user according to the subsequent steps of the method 300. For example, following step 302, the current plan may be the primary treatment plan by default.

The method 300 may include receiving, at step 308, inputs from the user that one or both of (a) configure a guide for a step of the current plan and (b) add text, measurement data, or other information defining the step of the current treatment plan. As an example, for implantation of a toric IOL, a step of the current treatment plan may include any of incision (which may include creating LRI), rhexis formation, removal of the crystalline lens, implantation, centration, alignment, and post-operative data collection. Configuring a guide may include configuring a guide according to the approaches described above with respect to **FIGS. 2A to 2E****.** The method 300 may include adding, at step 310, the guides and/or treatment plan from step 308 to the step of the current treatment plan.

The interface 100 may receive inputs from the user instructing a switch of the current treatment plan, such as from the primary treatment plan to a backup plan, from a backup plan to the primary treatment plan, or from a first backup plan to a second backup plan. Whichever plan is the new current plan following the switch may then have one more steps and guides added thereto (see steps 308 and 310, above) in response to inputs received through the interface 100.

If a switch is found, at step 312, to be instructed by the user and if the instruction is found, at step 316, to be an instruction to create a new backup plan, a new backup plan is created at step 318. The backup plan and the primary treatment plan may be part of a common data structure representing the same surgical procedure, the common data structure is referred to herein as the plan hierarchy. Following creation at step 318, the backup plan may then have one or more steps and one or more guides added thereto (see steps 308 and 310).

The method 300 may include evaluating, at step 320, whether the new backup plan is compatible with a parent plan, i.e., the primary treatment plan or previously created backup plan. In some instances, one or more steps or guides of the parent plan may be either identical to or compatible with corresponding one or more steps or guides of the new plan. "Compatible" as used herein therefore indicates that in the event of a switch between the first plan and the second plan, the one or more steps need not be repeated and/or the same one or more guides may be used. Whether the new plan created at step 318 is compatible with the current plan or other previously-created plan may be determined in response to an input from the user indicating compatibility. For example, the user may provide inputs indicating which steps and/or guides of the parent plan are compatible with the new plan.

If the new plan is determined at step 320 to be compatible, then, at step 322, the method 300 may include importing one or more previously defined steps of the parent plan into the new plan so that the user does not have to repeat entry of these steps into the new plan and may simply proceed with entry of subsequent steps to the new plan at subsequent iterations of step 310. Alternatively step 322 may include adding the new plan as a branch with respect to the parent plan. A "branch" may be an indication of compatibility between the parent plan and the new plan indicating that a step of the parent plan is compatible with a corresponding step of the new plan and the corresponding step of the new plan would not need to be performed in the event of a switch. As an example, a branch may indicate that an incision step of the parent plan is compatible with the incision step of the new plan such that upon switching from the parent plan to the new plan during a surgery, the incision step of the new plan may be omitted.

The method 300 may include switching, at step 324, the current plan to the plan indicated in the instruction from step 312, i.e., the new plan created at step 318 or an existing plan referenced by the instruction from step 312. The method 300 may then continue with addition of a new step to the current plan at step 310. Accordingly, the user may add steps to the newly created plan from step 318 or the existing plan.

The method 300 may continue until the user provides, at step 314, an input indicating that creation of the plan hierarchy is finished.

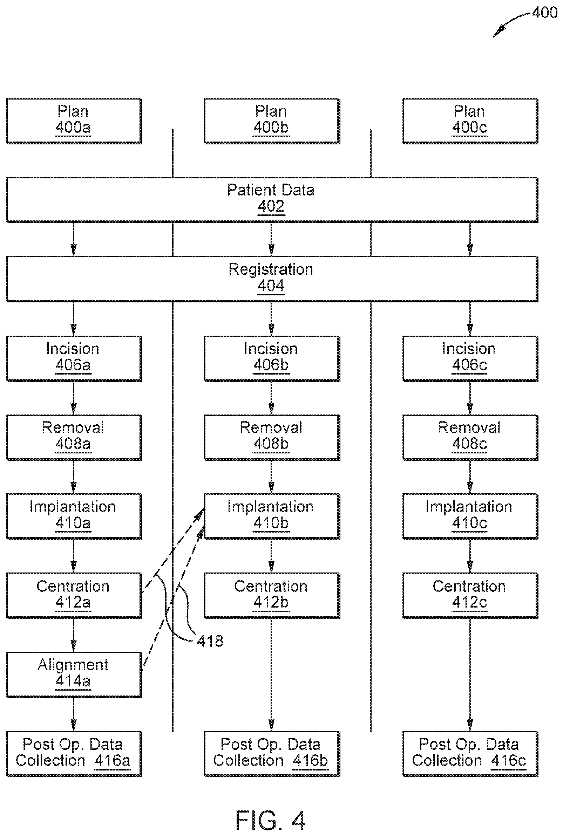

**FIG. 4** illustrates an example plan hierarchy 400 that may be created according to the method 300 or other method. The plan hierarchy 400 may include a plan 400a (e.g., the primary treatment plan) and one or more other plans 400b, 400c (e.g., backup plans). The plans 400a, 400b, 400c may have common data such as patient data 402 received at step 306 and a registration step 404 with respect to a common pre-operative image 102 received at step 304.

The registration step 404 may be performed by capturing a live image (e.g., intraoperative image) of the patient's eye and matching ocular anatomy, such as the unique patterns of blood vessels on the sclera and/or retina and/or the unique patterns of the limbus and/or iris represented in the image, to representations of corresponding ocular anatomy in the pre-operative image 102 to determine the orientation of the eye. The registration step 404 may include determining a transformation relating the orientation of the eye in the live image to the orientation of the eye in the pre-operative image 102. The locations, orientations, and/or size of guides defined in the treatment plans 400a, 400b, 400c may then be transformed according to the transformation and superimposed on the live image. The live image may be a frame of a plurality of frames of a video feed from an imaging device used during surgery. Accordingly, the registration step 404 may include initiating registration with respect to frames of the video feed, which may then be repeated throughout the surgery for every frame of the video feed or periodically, e.g., every N frames, where N is greater than 1, or in response to detection of movement of the patient's eye.

Each plan 400a, 400b, 400c may include other steps such as an incision step 406a, 406b, 406c, removal step 408a, 408b, 408c, implantation step 410a, 410b, 410c, centration step 412a, 412b, 412c, and a post-operative data collection step 416a, 416b, 416c. Some steps of a plan may not be present in other plans. For example, plan 400a may correspond to implantation of a toric IOL and therefore includes an alignment step 414a that is not present in other plans 400b, 400c.

The plan hierarchy 400 may include compatibility indicated by branches 418. For example, plan 400a may correspond to placement of a toric IOL whereas plan 400b corresponds to a backup plan in the case of rupture of the capsular bag such that a non-toric IOL must be used and sulcus placement is required. The plans 400a, 400b may be compatible with respect to the incision steps 406a, 406b and removal steps 408a, 408b. This is not to say that the incision steps 406a, 406b are identical. It may be that one or more incision guides 200 of plan 400a are different from the one or more incision guides 200 of plan 400b. However, in the event that a switch is made to plan 400b, the incisions from plan 400a may be used. In some cases, the incision steps may be compatible but not completely overlapping. For example, the incision step 406a may not include LRI whereas incision step 406b may require additional LRI for sulcus placement of a monofocal IOL. Accordingly, in the event of a switch from plan 400a to plan 400b, guides for placing the LRI may be displayed even when the incision step 406a was completed prior to the switch.

In another example scenario, plan 400c corresponds to a different type of IOL than plan 400a. In the event that a patient decides to switch from a toric IOL to a non-toric IOL, the surgeon may switch to plan 400c, for example, without delaying the surgery to create a new treatment plan. Where the incision steps 406a, 406c are compatible, such a switch could even be made during the surgery.

The plan hierarchy 400 illustrates a further benefit of the implementations described herein. The post-operative data that is collected for a surgery should correspond to the actual surgery performed. Upon switching between plans 400a, 400b, 400c, the post-operative data collection step 416a, 416b, 416c of whichever plan is the selected plan upon completion of the surgery may be performed. The post-operative data collection may include automatic collection of data from video data captured during the surgery and/or prompts for the surgeon to enter post-operative data. In this manner, post-operative care may be performed according to the plan actually implemented even when a change between plans 400a, 400b, 400c occurred during the surgery. In some implementations, the post-operative data collection step of plans that were started but not completed is omitted. For example, the post-operative data collection step 416a may be omitted when switching from plan 400a to plan 400b during the surgery.

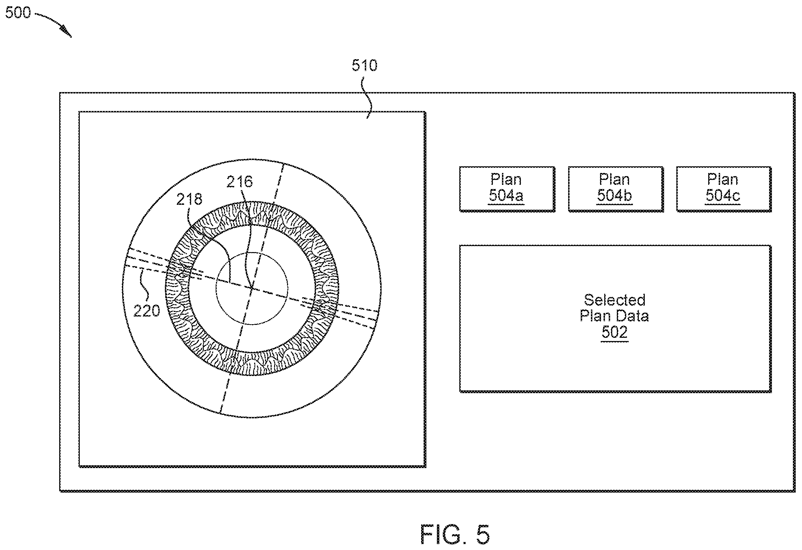

**FIG. 5** illustrates an example interface 500 for presenting plans 400a, 400b, 400c to a surgeon during a surgery. The interface 500 may include selected plan data 502. The selected plan data 502 may include data common to all the plans 400a, 400b, 400c, such as the patient data 402. The selected plan data 502 may include specific data corresponding to whichever of the plans 400a, 400b, 400c is a plan most recently selected by the surgeon for implementation. For example, for the plan 400a corresponding to placement of a toric IOL, the selected plan data 502 may include data defining a desired alignment of the toric IOL, a specification (e.g., refractive properties) of the toric IOL, or other data. For the plan 400b corresponding to bag rupture, the selected plan data 502 may include specifications of the non-toric IOL and describing sulcus placement thereof.

The interface 500 may include a live image 510 of the patient's eye captured during the surgery. For example, the live image 510 may include successively displayed frames of a video feed from a digital microscope having the patient's eye in the field of view thereof. The live image 510 may be registered as described above and have guides superimposed thereon according to whichever of the treatment plans 400a, 400b, 400c is the selected plan. For example, the IOL axis 218 and corresponding protractors 220 may be displayed for plan 400a corresponding to placement of a toric IOL.

In the case of a switch between plans 400a, 400b, 400c, some guides may correspond to a plan other than the selected plan. For example, suppose plans 400a, 400b have different incision guides 200 but a switch from plan 400a to plan 400b occurred after performing the incision step 406a. In that case, the incision guides 200 presented throughout implementation of the plan 400b may be those of the plan 400a.

In some scenarios, a switch between a first plan and a second plan of the plans 400a, 400b, 400c may include an automatic or predefined modification of one or more steps of the second plan. For example, an incision for a first plan may affect the post-operative astigmatism of the patient's eye. Accordingly, if the second plan includes placement of a toric IOL correcting for astigmatism, the refractive power and orientation of the toric IOL defined by the second plan may be recalculated to account for the incision step of the first plan. This calculation may be specified in the plan hierarchy 400 or may be automatically calculated in response to a post-incision switch from the first plan to the second plan.

The interface 500 may include plan elements 504a, 504b, 504c each corresponding to one of the plans 400a, 400b, 400c of the plan hierarchy 400. Each plan element 504a, 504b, 504c may have a label indicating the scenario to which the plan element 504a, 504b, 504c corresponds, such as "Toric IOL," "Bag Rupture," "Non-Toric IOL," or other label. Upon selection of a plan element 504a, 504b, 504c, the interface 500 may change the guides superimposed on the live image 510 and the selected plan data 502 of the plan 400a, 400b, 400c corresponding to the selected plan element 504a, 504b, 504c. For a switch from a first plan to a second plan of the plans 400a, 400b, 400c, the guides and plan data 502 may correspond to the first incompatible step of the second plan. For example, where the registration step 404, incision steps 406a, 406b, and removal steps 408a, 408b are compatible between plan 400a, 400b, the guides and plan data 502 displayed upon a switch from plan 400a to plan 400b following the removal step 408a may correspond to the implantation step 410b.

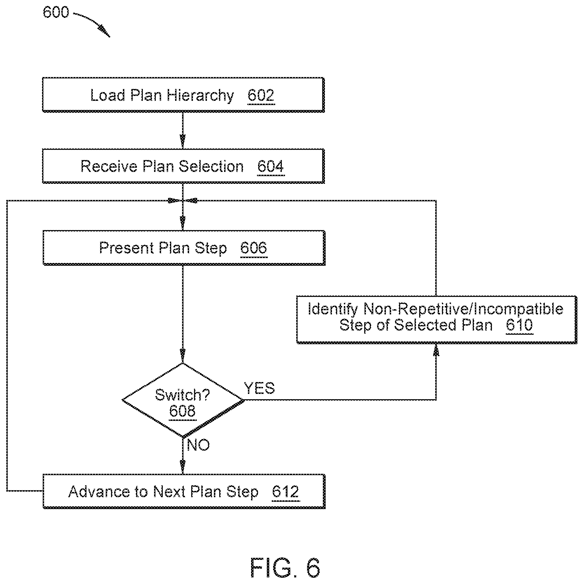

**FIG. 6** illustrates an example method 600 that may be performed by a computing device using inputs received through the interface 500 or other interface. The method 600 may include loading, at step 602, a plan hierarchy 400 including a plurality of plans 400a, 400b, 400c including a primary treatment plan and at least one backup plan. The method 600 may include receiving, at step 604, selection of a plan ("the selected plan") from the plan hierarchy. For example, the surgeon may select the primary treatment plan 400a using plan element 504a. In response, the method 600 includes presenting, at step 606, the first un-implemented step of the selected plan. Step 606 may include presenting guides and other data defined for the last un-implemented step of the selected plan. In the example of **FIG.** 4, presenting guides would include the registration step 404. A step of a treatment plan 400a, 400b, 400c may be deemed implemented either (a) as soon as the step is presented in the interface 500, (b) as soon as the surgeon advances from the step to the next step in the selected treatment plan or switches to a different treatment plan, or (c) in response to an input from the surgeon indicating that step has been implemented.

The method 600 may include determining, at step 608, whether the surgeon has switched plans. For example, the surgeon may commence performing plan 400a and then switch to plan 400b by selecting plan element 504b. If so, the method 600 may include setting the new plan selected by the surgeon as the selected plan and then identifying, at step 610, a first step of the selected plan that is either (a) non-repetitive with respect to implemented steps of the previous selected plan and (b) incompatible with respect to implemented steps of the previous selected plan. The step of the selected plan identified at step 610 may then be presented at step 606.

As used herein "non-repetitive" may be understood as not corresponding to a previously implemented identical or compatible step. For example, compatible incision steps 406a, 406b correspond to one another and can be repetitive with respect to one another. Suppose the registration step 404 of plan 400a was implemented prior to switching to plan 400b. In that case, performing the registration step 404 is repetitive and is not repeated. Instead, the incision step 406b would be performed as the first non-repetitive or incompatible step since the corresponding incision step 406a has not been performed and therefore step 406b is not repetitive. Suppose that the incision step 406a and removal step 408a have been performed prior to switching from plan 400a to plan 400b, the first non-repetitive or incompatible step of plan 400b would therefore be the implantation step 410b. Likewise, if any of the implantation step 410a, centration step 412a, and alignment steps 414a have been performed for the plan 400a prior to switching to plan 400b due to capsular bag rupture, the first non-repetitive or incompatible step of plan 400b would be implantation step 410b of a non-toric IOL.

In the absence of a switching between plans, following presentation of a step for the selected plan at step 606, the interface 500 may be advanced, at step 612, to the next step of the selected plan. Advancing may be performed in response to an input from the surgeon instructing the interface 500 to advance to the next step.

The method 600 may continue until the last step of the selected plan, e.g., a post-operative-data collection step 416a, 416b, 416b for the selected plain is implemented.

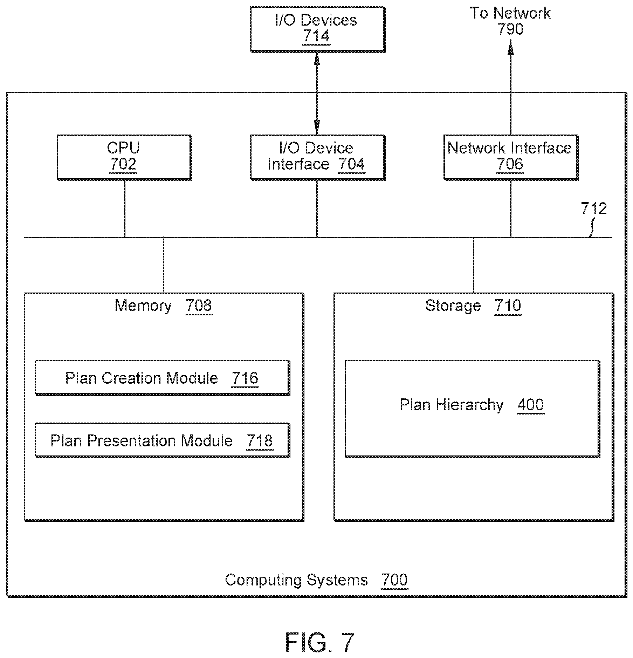

**FIG. 7** illustrates an example computing system 700 that implements, at least partly, one or more functionalities described herein in response to inputs to the interface 100 and/or interface 500. The computing system 700 may also implement the methods 300 and/or 600. The computing system 700 may be integrated with an imaging device, such as a digital microscope, or be a separate computing device receiving images of a patient's eye from the imaging device. In practice, the computing device 700 used to implement the method 300 and present the interface 100 may be different from the computing device 700 used to implement the method 600 and present the interface 500. For example, the computing device 700 used to implement the method 300 and present the interface 100 may be a general purpose computing device whereas the computing device 700 used to implement the method 600 and present the interface 500 may be part of a surgical imaging system.

As shown, computing system 700 includes a central processing unit (CPU) 702, one or more I/O device interfaces 704, which may allow for the connection of various I/O devices 714 (e.g., keyboards, displays, mouse devices, pen input, etc.) to computing system 700, network interface 706 through which computing system 700 is connected to network 790 (which may be a local network, an intranet, the internet, or any other group of computing systems communicatively connected to each other, as described in relation to **FIG.** 1), a memory 708, storage 710, and an interconnect 712.

In cases where computing system 700 is an imaging system, such as a digital microscope, computing system 700 may further include one or more optical components for obtaining ophthalmic imaging of a patient's eye as well as any other components known to one of ordinary skill in the art. In cases where computing system 700 is a surgical microscope, computing system 700 may further include many other components known to one of ordinary skill in the art to perform the ophthalmic surgeries described herein as known to one of ordinary skill in the art.

CPU 702 may retrieve and execute programming instructions stored in the memory 708. Similarly, CPU 702 may retrieve and store application data residing in the memory 708. The interconnect 712 transmits programming instructions and application data, among CPU 702, I/O device interface 704, network interface 706, memory 708, and storage 710. CPU 702 is included to be representative of a single CPU, multiple CPUs, a single CPU having multiple processing cores, and the like.

Memory 708 is representative of a volatile memory, such as a random access memory, and/or a nonvolatile memory, such as nonvolatile random access memory, phase change random access memory, or the like. As shown, memory 708 may store a plan creation module 716 including executable code instructing the CPU 702 to implement one or both of the interface 100 and the method 300. The memory 708 may store a plan presentation module 718 including executable code instructing the CPU 702 to implement one or both of the interface 500 and the method 600.

Storage 710 may be non-volatile memory, such as a disk drive, solid state drive, or a collection of storage devices distributed across multiple storage systems. Storage 710 may optionally store a plan hierarchy 400 as described herein.

### Additional Considerations

The preceding description is provided to enable any person skilled in the art to practice the various embodiments described herein. Various modifications to these embodiments will be readily apparent to those skilled in the art, and the generic principles defined herein may be applied to other embodiments. For example, changes may be made in the function and arrangement of elements discussed without departing from the scope of the disclosure. Various examples may omit, substitute, or add various procedures or components as appropriate. Also, features described with respect to some examples may be combined in some other examples. For example, an apparatus may be implemented or a method may be practiced using any number of the aspects set forth herein. In addition, the scope of the disclosure is intended to cover such an apparatus or method that is practiced using other structure, functionality, or structure and functionality in addition to, or other than, the various aspects of the disclosure set forth herein. It should be understood that any aspect of the disclosure disclosed herein may be embodied by one or more elements of a claim.

As used herein, a phrase referring to "at least one of" a list of items refers to any combination of those items, including single members. As an example, "at least one of: a, b, or c" is intended to cover a, b, c, a-b, a-c, b-c, and a-b-c, as well as any combination with multiples of the same element (e.g., a-a, a-a-a, a-a-b, a-a-c, a-b-b, a-c-c, b-b, b-b-b, b-b-c, c-c, and c-c-c or any other ordering of a, b, and c).

As used herein, the term "determining" encompasses a wide variety of actions. For example, "determining" may include calculating, computing, processing, deriving, investigating, looking up (e.g., looking up in a table, a database or another data structure), ascertaining and the like. Also, "determining" may include receiving (e.g., receiving information), accessing (e.g., accessing data in a memory) and the like. Also, "determining" may include resolving, selecting, choosing, establishing and the like.

The methods disclosed herein comprise one or more steps or actions for achieving the methods. The method steps and/or actions may be interchanged with one another without departing from the scope of the claims. In other words, unless a specific order of steps or actions is specified, the order and/or use of specific steps and/or actions may be modified without departing from the scope of the claims. Further, the various operations of methods described above may be performed by any suitable means capable of performing the corresponding functions. The means may include various hardware and/or software component(s) and/or module(s), including, but not limited to a circuit, an application specific integrated circuit (ASIC), or processor. Generally, where there are operations illustrated in figures, those operations may have corresponding counterpart means-plus-function components with similar numbering.

The various illustrative logical blocks, modules and circuits described in connection with the present disclosure may be implemented or performed with a general purpose processor, a digital signal processor (DSP), an application specific integrated circuit (ASIC), a field programmable gate array (FPGA) or other programmable logic device (PLD), discrete gate or transistor logic, discrete hardware components, or any combination thereof designed to perform the functions described herein. A general-purpose processor may be a microprocessor, but in the alternative, the processor may be any commercially available processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

A processing system may be implemented with a bus architecture. The bus may include any number of interconnecting buses and bridges depending on the specific application of the processing system and the overall design constraints. The bus may link together various circuits including a processor, machine-readable media, and input/output devices, among others. A user interface (e.g., keypad, display, mouse, joystick, etc.) may also be connected to the bus. The bus may also link various other circuits such as timing sources, peripherals, voltage regulators, power management circuits, and the like, which are well known in the art, and therefore, will not be described any further. The processor may be implemented with one or more general-purpose and/or special-purpose processors. Examples include microprocessors, microcontrollers, DSP processors, and other circuitry that can execute software. Those skilled in the art will recognize how best to implement the described functionality for the processing system depending on the particular application and the overall design constraints imposed on the overall system.

If implemented in software, the functions may be stored or transmitted over as one or more instructions or code on a computer-readable medium. Software shall be construed broadly to mean instructions, data, or any combination thereof, whether referred to as software, firmware, middleware, microcode, hardware description language, or otherwise. Computer-readable media include both computer storage media and communication media, such as any medium that facilitates transfer of a computer program from one place to another. The processor may be responsible for managing the bus and general processing, including the execution of software modules stored on the computer-readable storage media. A computer-readable storage medium may be coupled to a processor such that the processor can read information from, and write information to, the storage medium. In the alternative, the storage medium may be integral to the processor. By way of example, the computer-readable media may include a transmission line, a carrier wave modulated by data, and/or a computer readable storage medium with instructions stored thereon separate from the wireless node, all of which may be accessed by the processor through the bus interface. Alternatively, or in addition, the computer-readable media, or any portion thereof, may be integrated into the processor, such as the case may be with cache and/or general register files. Examples of machine-readable storage media may include, by way of example, RAM (Random Access Memory), flash memory, ROM (Read Only Memory), PROM (Programmable Read-Only Memory), EPROM (Erasable Programmable Read-Only Memory), EEPROM (Electrically Erasable Programmable Read-Only Memory), registers, magnetic disks, optical disks, hard drives, or any other suitable storage medium, or any combination thereof. The machine-readable media may be embodied in a computer-program product.

A software module may comprise a single instruction, or many instructions, and may be distributed over several different code segments, among different programs, and across multiple storage media. The computer-readable media may comprise a number of software modules. The software modules include instructions that, when executed by an apparatus such as a processor, cause the processing system to perform various functions. **The** software modules may include a transmission module and a receiving module. Each software module may reside in a single storage device or be distributed across multiple storage devices. By way of example, a software module may be loaded into RAM from a hard drive when a triggering event occurs. During execution of the software module, the processor may load some of the instructions into cache to increase access speed. One or more cache lines may then be loaded into a general register file for execution by the processor. When referring to the functionality of a software module, it will be understood that such functionality is implemented by the processor when executing instructions from that software module.

## Claims

1. A method for guiding ophthalmic surgery comprising:
loading, by a computing device (101), two or more ophthalmic surgical treatment plans (400) having a common pre-operative image (102) of a patient, the two or more treatment plans being different from one another;
receiving, by the computing device (101), one or more live images (103) of an eye of the patient;
initiating, by the computing device (101), registration of the one or more live images with respect to the pre-operative image (104);
initiating, by the computing device (101), on a display device (105) coupled to the computing device, presentation of one or more first steps of a first treatment plan of the two or more treatment plans, the one or more first steps defining superimposition of one or more first guides (106) on the one or more live images (103);
receiving, by the computing device (101), an instruction to switch to a second plan of the two or more treatment plans; in response to the instruction, initiating, by the computing device (101), on the display device (105), presentation of one or more second steps of a second plan of the two or more treatment plans, the one or more second steps defining superimposition of one or more second guides (108) on the one or more live images, the one or more second guides being different from the one or more first guides;
further comprising:
(a) determining, by the computing device (101), that a portion of the one or more second steps are compatible with a portion of the one or more first steps presented on the display device prior to receiving the instruction to switch to the second plan; and
in response to (a), omitting presentation of the portion of the one or more second steps.

2. The method of claim 1, further comprising:
(b) identifying, by the computing device (101), an incompatible step of the one or more second steps that is incompatible with the one or more first steps; and
in response to (b), initiating, by the computing device, presentation of the one or more second steps with the incompatible step.

3. The method of claim 1, wherein the first treatment plan corresponds to placement of a toric intraocular lens, IOL, (112) and the second treatment plan corresponds to placement of a non-toric IOL (113).

4. The method of claim 1, wherein the one or more first steps include an incision step, a removal step corresponding to removal of a crystalline lens of the eye of the patient, a centration step of an intraocular lens (IOL), and an alignment step of an IOL axis of the IOL.

5. The method of claim 1, wherein the one or more first steps comprise a plurality of first steps, the method further comprising:
receiving the instruction to switch to the second plan of the two or more treatment plans prior to completing presentation of all steps of the plurality of first step; and
performing, by the computing device (101), a second post-operative data collection step included in the one or more second steps and omitting performance of a first post-operative data collection step included in the one or more first steps.

6. The method of claim 1, wherein the one or more first guides include one or more of:
an incision indicator (122);
a rhexis guide (123);
a limbal release incision guide, LRI, (124);
a centration guide (125); or
an intraocular lens, IOL, axis (126).

7. The method of claim 1, further comprising, initiating presentation of the one or more second steps without again initiating registration of the live video (127) with respect to the pre-operative image (102).

8. A system for guiding ophthalmic surgery comprising:
one or more processing devices (128);
one or more memory devices (129) coupled to the one or more processing devices and storing executable code that, when executed by the one or more processing devices, causes the one or more processing devices to:
load two or more ophthalmic surgical treatment plans having a common pre-operative image (102) of a patient, the two or more treatment plans being different from one another, the two or more treatment plans;
receive a live video (127) of an eye of the patient;
initiate registration of the live video (127) with respect to the pre-operative image (102);
initiate presentation of one or more first steps of a first treatment plan of the two or more treatment plans on a display device (105), the one or more first steps defining superimposition of one or more first guides (106) on the live video;
receive an instruction to switch to a second plan of the two or more treatment plans; and
in response to the instruction, initiating, presentation of one or more second steps of a second plan of the two or more treatment plans on the display device (105), the one or more second steps defining superimposition of one or more second guides (108) on the live video, the one or more second guides being different from the one or more first guides.
wherein the executable code when executed by the one or more processing devices further causes the one or more processing devices to:
evaluate whether (a) a portion of the one or more second steps are compatible with a portion of the one or more first steps presented on the display device prior to receiving the instruction to switch to the second plan; and
if (a) is true, omit presentation of the portion of the one or more second steps.

9. The system of claim 8, wherein the executable code when executed by the one or more processing devices further causes the one or more processing devices to:
identify an incompatible step of the one or more second steps that is incompatible with the one or more first steps; and
initiate presentation of the one or more second steps with the incompatible step.

10. The system of claim 9, wherein the first treatment plan corresponds to placement of a toric intraocular lens, IOL, (112) in a capsular bag of the eye of the patient and the second treatment plan corresponds to sulcus placement of a non-toric IOL (113).

11. The system of claim 9,
wherein the one or more first steps comprise a plurality of first steps; and
wherein the executable code when executed by the one or more processing devices further causes the one or more processing devices to receive the instruction to switch to the second plan of the two or more treatment plans prior to completing presentation of all steps of the plurality of first steps.

12. The system of claim 8, wherein the executable code when executed by the one or more processing devices further causes the one or more processing devices to:
perform a second post-operative data collection step included in the one or more second steps; and
refrain from performing a first post-operative data collection step included in the one or more first steps.

13. The system of claim 9, wherein the executable code when executed by the one or more processing devices further causes the one or more processing devices to:
initiate presentation of the one or more second steps without again initiating registration of the live video (127) with respect to the pre-operative image (102).

## Patentansprüche

1. Verfahren zum Führen von ophthalmischer Chirurgie, umfassend:
Laden, durch eine Rechenvorrichtung (101), von zwei oder mehr ophthalmisch-chirurgischen Behandlungsplänen (400) mit einem gemeinsamen präoperativen Bild (102) eines Patienten, wobei die zwei oder mehr Behandlungspläne voneinander verschieden sind;
Empfangen, durch die Rechenvorrichtung (101), eines oder mehrerer Live-Bilder (103) eines Auges des Patienten;
Auslösen, durch die Rechenvorrichtung (101), von Abgleichen des einen oder der mehreren Live-Bilder bezogen auf das präoperative Bild (104);
Auslösen, durch die Rechenvorrichtung (101), auf einer an die Rechenvorrichtung gekoppelten Anzeigevorrichtung (105) einen oder mehrere erste Schritte eines ersten Behandlungsplans der zwei oder mehr Behandlungspläne darzustellen, wobei der eine oder die mehreren ersten Schritte Überlagerung eines oder mehrerer erster Führungselemente (106) auf dem einen oder den mehreren Live-Bildern (103) definieren;
Empfangen, durch die Rechenvorrichtung (101), einer Anweisung zum Wechseln zu einem zweiten Plan der zwei oder mehr Behandlungspläne; als Reaktion auf die Anweisung Auslösen, durch die Rechenvorrichtung (101), auf der Anzeigevorrichtung (105) einen oder mehrere zweite Schritte eines zweiten Plans der zwei oder mehr Behandlungspläne darzustellen, wobei der eine oder die mehreren zweiten Schritte Überlagerung eines oder mehrerer zweiter Führungselemente (108) auf dem einen oder den mehreren Live-Bildern definieren, wobei sich das eine oder die mehreren zweiten Führungselemente von dem einen oder den mehreren ersten Führungselementen unterscheiden;
ferner umfassend:
(a) Bestimmen, durch die Rechenvorrichtung (101), dass ein Teil des einen oder der mehreren zweiten Schritte mit einem Teil des einen oder der mehreren ersten Schritte, die auf der Anzeigevorrichtung dargestellt werden, vereinbar ist, bevor die Anweisung zum Wechseln zu dem zweiten Plan empfangen wird; und
als Reaktion auf (a) Weglassen von Darstellen des Teils des einen oder der mehreren zweiten Schritte.

2. Verfahren nach Anspruch 1, ferner umfassend:
(b) Identifizieren, durch die Rechenvorrichtung (101), eines unvereinbaren Schritts des einen oder der mehreren zweiten Schritte, der mit dem einen oder den mehreren ersten Schritten unvereinbar ist; und
als Reaktion auf (b) Auslösen, durch die Rechenvorrichtung, von Darstellung des einen oder der mehreren zweiten Schritte mit dem unvereinbaren Schritt.

3. Verfahren nach Anspruch 1, wobei der erste Behandlungsplan Platzierung einer torischen Intraokularlinse, IOL, (112) entspricht und der zweite Behandlungsplan Platzierung einer nicht-torischen IOL (113) entspricht.

4. Verfahren nach Anspruch 1, wobei der eine oder die mehreren ersten Schritte einen Inzisionsschritt, einen Entfernungsschritt, der Entfernen einer Linse des Auges des Patienten entspricht, einen Zentrierungsschritt einer Intraokularlinse (IOL) und einen Ausrichtungsschritt einer IOL-Achse der IOL umfassen.

5. Verfahren nach Anspruch 1, wobei der eine oder die mehreren ersten Schritte mehrere erste Schritte umfassen, wobei das Verfahren ferner umfasst:
Empfangen der Anweisung zum Wechseln zu dem zweiten Plan der zwei oder mehr Behandlungspläne vor Abschluss der Darstellung aller Schritte der mehreren ersten Schritte; und
Durchführen, durch die Rechenvorrichtung (101), eines zweiten postoperativen Datensammelschritts, der in dem einen oder den mehreren zweiten Schritten enthalten ist, und Weglassen der Durchführung eines ersten postoperativen Datensammelschritts, der in dem einen oder den mehreren ersten Schritten enthalten ist.

6. Verfahren nach Anspruch 1, wobei das eine oder die mehreren ersten Führungselemente eines oder mehrere umfassen von:
einem Inzisionsindikator (122);
einer Rhexis-Führung (123);
einer limbale-Freisetzung-Inzisionsführung, LRI, (124);
einer Zentrierungsführung (125); oder
einer Intraokularlinsen(IOL)-Achse (126).

7. Verfahren nach Anspruch 1, ferner umfassend Auslösen von Darstellung des einen oder der mehreren zweiten Schritte ohne erneutes Auslösen von Abgleich des Live-Videos (127) bezogen auf das präoperative Bild (102).

8. System zum Führen von ophthalmischer Chirurgie, umfassend:
eine oder mehrere Verarbeitungsvorrichtungen (128);
eine oder mehrere Speichervorrichtungen (129), die an die eine oder mehreren Verarbeitungsvorrichtungen gekoppelt sind und ausführbaren Code speichern, der bei Ausführung durch die eine oder mehreren Verarbeitungsvorrichtungen die eine oder mehreren Verarbeitungsvorrichtungen veranlasst zum:
Laden von zwei oder mehr ophthalmisch-chirurgischen Behandlungsplänen mit einem gemeinsamen präoperativen Bild (102) eines Patienten, wobei die zwei oder mehr Behandlungspläne voneinander verschieden sind, die zwei oder mehr Behandlungspläne;
Empfangen eines Live-Videos (127) eines Auges des Patienten;
Auslösen von Abgleichen des Live-Videos (127) bezogen auf das präoperative Bild (102);
Auslösen von Darstellung eines oder mehrerer erster Schritte eines ersten Behandlungsplans der zwei oder mehr Behandlungspläne auf einer Anzeigevorrichtung (105), wobei der eine oder die mehreren ersten Schritte Überlagerung eines oder mehrerer erster Führungselemente (106) auf dem Live-Video definieren;
Empfangen einer Anweisung zum Wechseln zu einem zweiten Plan der zwei oder mehr Behandlungspläne; und
als Reaktion auf die Anweisung Auslösen von Darstellung eines oder mehrerer zweiter Schritte eines zweiten Plans der zwei oder mehr Behandlungspläne auf der Anzeigevorrichtung (105), wobei der eine oder die mehreren zweiten Schritte Überlagern eines oder mehrerer zweiter Führungselemente (108) auf dem Live-Video definieren, wobei sich das eine oder die mehreren zweiten Führungselemente von dem einen oder den mehreren ersten Führungselementen unterscheiden;
wobei der ausführbare Code bei Ausführung durch die eine oder mehreren Verarbeitungsvorrichtungen ferner die eine oder mehreren Verarbeitungsvorrichtungen veranlasst zum:
Beurteilen, ob (a) ein Teil des einen oder der mehreren zweiten Schritte mit einem Teil des einen oder der mehreren ersten Schritte, die auf der Anzeigevorrichtung dargestellt werden, vereinbar ist, bevor die Anweisung zum Wechseln zu dem zweiten Plan empfangen wird; und
wenn (a) zutrifft, Weglassen von Darstellen des Teils des einen oder der mehreren zweiten Schritte.

9. System nach Anspruch 8, wobei der ausführbare Code bei Ausführung durch die eine oder mehreren Verarbeitungsvorrichtungen ferner die eine oder mehreren Verarbeitungsvorrichtungen veranlasst zum:
Identifizieren eines unvereinbaren Schritts des einen oder der mehreren zweiten Schritte, der mit dem einen oder den mehreren ersten Schritten unvereinbar ist; und
Auslösen von Darstellung des einen oder der mehreren zweiten Schritte mit dem unvereinbaren Schritt.

10. System nach Anspruch 9, wobei der erste Behandlungsplan Platzierung einer torischen Intraokularlinse, IOL, (112) in einem Kapselsack des Auges des Patienten entspricht und der zweite Behandlungsplan Sulcus-Platzierung einer nicht-torischen IOL (113) entspricht.

11. System nach Anspruch 9, wobei der eine oder die mehreren ersten Schritte mehrere erste Schritte umfassen; und
wobei der ausführbare Code bei Ausführung durch die eine oder mehreren Verarbeitungsvorrichtungen ferner die eine oder mehreren Verarbeitungsvorrichtungen dazu veranlasst, die Anweisung empfangen, zu dem zweiten Plan der zwei oder mehr Behandlungspläne zu wechseln, bevor die Darstellung aller Schritte der mehreren ersten Schritte abgeschlossen wird.

12. System nach Anspruch 8, wobei der ausführbare Code bei Ausführung durch die eine oder mehreren Verarbeitungsvorrichtungen ferner die eine oder mehreren Verarbeitungsvorrichtungen veranlasst zum:
Durchführen eines zweiten postoperativen Datensammelschritts, der in dem einen oder den mehreren zweiten Schritten enthalten ist; und
Unterlassen der Durchführung eines ersten postoperativen Datensammelschritts, der in dem einen oder den mehreren ersten Schritten enthalten ist.

13. System nach Anspruch 9, wobei der ausführbare Code bei Ausführung durch die eine oder mehreren Verarbeitungsvorrichtungen ferner die eine oder mehreren Verarbeitungsvorrichtungen veranlasst zum:
Auslösen von Darstellung des einen oder der mehreren zweiten Schritte ohne erneutes Auslösen von Abgleich des Live-Videos (127) bezogen auf das präoperative Bild (102).

## Revendications

1. Procédé destiné au guidage de chirurgie ophtalmique comprenant :
le chargement, par un dispositif informatique (101), de deux plans de traitement chirurgical ophtalmique ou plus (400) ayant une image préopératoire commune (102) d'un patient, les deux plans de traitement ou plus étant différents l'un de l'autre ;
la réception, par le dispositif informatique (101), d'une ou plusieurs images en direct (103) d'un œil du patient ; l'initiation, par le dispositif informatique (101), de l'enregistrement de la ou des images en direct par rapport à l'image préopératoire (104) ;
l'initiation, par le dispositif informatique (101), sur un dispositif d'affichage (105) couplé au dispositif informatique, de la présentation d'une ou plusieurs premières étapes d'un premier plan de traitement des deux plans de traitement ou plus, la ou les premières étapes définissant la superposition d'un ou plusieurs premiers guides (106) sur la ou les images en direct (103) ;
la réception, par le dispositif informatique (101), d'une instruction de passer à un second plan des deux plans de traitement ou plus ; en réponse à l'instruction, d'initiation, par le dispositif informatique (101), sur le dispositif d'affichage (105), de la présentation d'une ou plusieurs secondes étapes d'un second plan des deux plans de traitement ou plus, la ou les secondes étapes définissant la superposition d'un ou plusieurs seconds guides (108) sur la ou les images en direct, le ou les seconds guides étant différents du ou des premiers guides ;
comprenant en outre :
(a) la détermination, par le dispositif informatique (101), qu'une partie de la ou des secondes étapes sont compatibles avec une partie de la ou des premières étapes présentées sur le dispositif d'affichage avant de recevoir l'instruction de passer au second plan ; et
en réponse à (a), l'omission de la présentation de la partie de la ou des secondes étapes.

2. Procédé selon la revendication 1, comprenant en outre :
(b) l'identification, par le dispositif informatique (101), d'une étape incompatible de la ou des secondes étapes qui est incompatible avec la ou les premières étapes ; et
en réponse à (b), l'initiation, par le dispositif informatique, de la présentation de la ou des secondes étapes avec l'étape incompatible.

3. Procédé selon la revendication 1, le premier plan de traitement correspondant au placement d'une lentille intraoculaire torique, LIO, (112) et le second plan de traitement correspondant au placement d'une LIO non torique (113).

4. Procédé selon la revendication 1, la ou les premières étapes comprenant une étape d'incision, une étape de retrait correspondant au retrait d'une lentille cristalline de l'œil du patient, une étape de centrage d'une lentille intraoculaire (LIO), et une étape d'alignement d'un axe de LIO de la LIO.

5. Procédé selon la revendication 1, la ou les premières étapes comprenant une pluralité de premières étapes, le procédé comprenant en outre :
la réception de l'instruction de passer au second plan des deux plans de traitement ou plus avant de terminer la présentation de toutes les étapes de la pluralité de premières étapes ; et
la réalisation, par le dispositif informatique (101), d'une seconde étape de collecte de données post-opératoires comprise dans la ou les secondes étapes et l'omission de la réalisation d'une première étape de collecte de données post-opératoires comprise dans la ou les premières étapes.

6. Procédé selon la revendication 1, le ou les premiers guides incluent un ou plusieurs éléments parmi :
un indicateur d'incision (122) ;
un guide de rhexis (123) ;
un guide d'incision limbique relaxante, LRI, (124) ;
un guide de centrage (125) ; ou
un axe (126) de lentille intraoculaire, LIO.

7. Procédé selon la revendication 1, comprenant en outre l'initiation de la présentation de la ou des secondes étapes sans initier à nouveau l'enregistrement de la vidéo en direct (127) par rapport à l'image pré-opératoire (102).

8. Système destiné au guidage de chirurgie ophtalmique comprenant :
un ou plusieurs dispositifs de traitement (128) ;
un ou plusieurs dispositifs de mémoire (129) couplés au ou aux dispositifs de traitement et stockant du code exécutable qui, lorsqu'il est exécuté par le ou les dispositifs de traitement, amène le ou les dispositifs de traitement à :
charger deux plans de traitement chirurgical ophtalmique ou plus ayant une image préopératoire commune (102) d'un patient, les deux plans de traitement ou plus étant différents l'un de l'autre, des deux plans de traitement ou plus ;
recevoir une vidéo en direct (127) d'un œil du patient ;
initier l'enregistrement de la vidéo en direct (127) par rapport à l'image préopératoire (102) ;
initier la présentation d'une ou plusieurs premières étapes d'un premier plan de traitement des deux plans de traitement ou plus sur un dispositif d'affichage (105),
la ou les premières étapes définissant la superposition d'un ou plusieurs premiers guides (106) sur la vidéo en direct ;
recevoir une instruction pour passer à un second plan des deux plans de traitement ou plus ; et
en réponse à l'instruction, initiation, de la présentation d'une ou plusieurs secondes étapes d'un second plan des deux plans de traitement ou plus sur le dispositif d'affichage (105), la ou les secondes étapes définissant la superposition d'un ou plusieurs seconds guides (108) sur la vidéo en direct, le ou les seconds guides étant différents du ou des premiers guides.
le code exécutable lorsqu'il est exécuté par le ou les dispositifs de traitement amenant en outre le ou les dispositifs de traitement à :
évaluer si (a) une partie de la ou des secondes étapes sont compatibles avec une partie de la ou des premières étapes présentées sur le dispositif d'affichage préalablement à la réception de l'instruction de passage au second plan ; et
si (a) est vrai, omettre la présentation de la partie de la ou des secondes étapes.

9. Système selon la revendication 8, le code exécutable lorsqu'il est exécuté par le ou les dispositifs de traitement amenant en outre le ou les dispositifs de traitement à :
identifier une étape incompatible de la ou des secondes étapes qui est incompatible avec la ou les premières étapes ; et
initier la présentation de la ou des secondes étapes avec l'étape incompatible.

10. Système selon la revendication 9, le premier plan de traitement correspondant à la mise en place d'une lentille intraoculaire torique, LIO (112) dans une poche capsulaire de l'œil du patient et le second plan de traitement correspondant à la mise en place de sulcus d'une LIO non torique (113).

11. Système selon la revendication 9, la ou les premières étapes comprenant une pluralité de premières étapes ; et
le code exécutable lorsqu'il est exécuté par le ou les dispositifs de traitement amenant en outre le ou les dispositifs de traitement à recevoir l'instruction de passer au second plan des deux plans de traitement ou plus avant de terminer la présentation de toutes les étapes de la pluralité de premières étapes.

12. Système selon la revendication 8, le code exécutable lorsqu'il est exécuté par le ou les dispositifs de traitement amenant en outre le ou les dispositifs de traitement à :
réaliser une seconde étape de collecte de données post-opératoires incluse dans la ou les secondes étapes ; et
s'abstenir de réaliser une première étape de collecte de données post-opératoires incluse dans la ou les premières étapes.

13. Système selon la revendication 9, le code exécutable lorsqu'il est exécuté par le ou les dispositifs de traitement amenant en outre le ou les dispositifs de traitement à :
initier la présentation de la ou des secondes étapes sans initier à nouveau l'enregistrement de la vidéo en direct (127) par rapport à l'image pré-opératoire (102).
